# EUROPEAN PATENT SPECIFICATION

(11) **EP 3 738 584 B1**
(45) Date of publication and mention of the grant of the patent: **29.06.2022**
(21) Application number: 20174336.6
(22) Date of filing: 13.05.2020
(51) Int. Cl.: A61K 9/16, A61K 9/20, A61K 31/55, A61K 9/48, A61P 25/08

(54) **GRANULATE CONTAINING ESLICARBAZEPINE ACETATE, ITS PRODUCTION, PHARMACEUTICAL PREPARATIONS CONTAINING IT, AND THEIR USE**
GRANULATE ENTHALTEND ESLICARBAZEPINACETAT, SOWIE DEREN HERSTELLUNG, PHARMAZEUTISCHE ZUSAMMENSETZUNGEN UND VERWENDUNG
GRANULES CONTENANT DE L'ACETATE D'ESLICARBAZEPINE, LEUR PRODUCTION, PRÉPARATION ET USAGE

(30) Priority: 14.05.2019 HU 1900153
(43) Date of publication of application: 18.11.2020
(73) Proprietor: MEDITOP Gyógyszeripari Korlátolt Felelösségü Társaság, 2097 Pilisborosjenö (HU)
(72) Inventor: Fekete, Pál, 1141 Budapest (HU); Bajdik, János, 2143 Kistarcsa (HU); Gál, Lívia, 2000 Szentendre (HU); Öszi, Zsolt, 2220 Vecsés (HU)
(74) Representative: Kovári, Zoltán

(56) References cited:
- WO-A1-2009/054743
- WO-A1-2017/103876
- Anonymous: "Low-Substituted Hydroxypropyl Cellulose NF L-HPC", , 1 August 2005 (2005-08-01), XP055434172, Retrieved from the Internet: URL:http://www.elementoorganika.ru/files/l hpc.pdf [retrieved on 2017-12-12]
- Anonymous: "NBD Grades (New Binder Disintegrant)", Shin Etsu - Brochure, 1 October 2011 (2011-10-01), XP055702130, Retrieved from the Internet: URL:http://www.dalian-diligence.com/images /NBD-EN.pdf [retrieved on 2020-06-08]
- ROWE R C ET AL: "Handbook of pharmaceutical excipients, Hydroxypropyl Cellulose, Low-substituted", 1 January 2006 (2006-01-01), HANDBOOK OF PHARMACEUTICAL EXCIPIENTS, PHARMACEUTICAL PRESS [U.A.], LONDON [U.A.], PAGE(S) 341 - 343, XP002547079, ISBN: 978-1-58212-058-4 * the whole document * * section "6 Functional Category" *

## Description

### Field of the invention

The object of the invention relates to granules containing eslicarbazepine acetate and L-HPC, a method for producing such granules, other pharmaceutical preparations produced from such granules, and to the use of such granules and pharmaceutical preparations.

### State of the art

Eslicarbazepine acetate is an active pharmaceutical ingredient for treating partial epileptic seizures. At present the company BIAL - Portela & Ca, S.A., the developer of the active ingredient, markets a pharmaceutical product containing eslicarbazepine acetate in the European Union under the name Zebinex (EMEA/H/C/000988, EPAR - Product information).

Eslicarbazepine acetate received its name from its active metabolite, (S)-licarbazepine, where the "S" refers to the spatial position of the oxygen atom bound to the central (azepanic) ring.

The main identifiers of eslicarbazepine acetate are the following:
INN name: eslicarbazepine acetate;
Abbreviated name: ESL;
IUPAC-name: (*S*)-10-acetoxy-10,11-dihydro-5*H-*dibenz[*b,f*]azepine-5-carboxamide;
Structural formula:

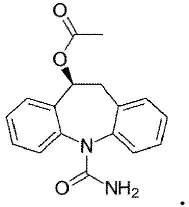

According to product information No. EMEA/H/C/000988 the exact mechanism of action of the molecule is unknown. *In vitro* electrophysiology tests demonstrate that both eslicarbazepine acetate and its metabolites stabilise the inactivated state of the voltage-dependant Na⁺ channels, thereby preventing them from turning again into activated state, and as a consequence they maintain repetitive neuronal discharge. Eslicarbazepine acetate is actually a prodrug that metabolises into S-licarbazepine in the body during absorption, which is what actually elicits the effect. The elimination half-life of (S)-licarbazepine is 13 to 20 hours, which makes it possible to administer the preparation once a day. Nevertheless, the daily dose of eslicarbazepine acetate is relatively high, 800 to 1600 mg.

Due to the unfavourable physical characteristics of the active ingredient (small grain size, low bulk density, bad compressibility and solubility, high dose) formulating eslicarbazepine acetate into an oral pharmaceutical preparation, primarily a tablet or capsule, is a difficult task.

However, it is important to be able to get the active ingredient into the body as quickly as possible in order to elicit the effect that treats partial epileptic seizures, therefore immediate release tablets are preferred in the case of oral preparations containing eslicarbazepine acetate. Patent application No. US 2009/0110722A1 discloses licarbazepine, particularly eslicarbazepine formulations that contain at least one excipient. These excipients may be diluents/fillers, binders, disintegrants, glidants and lubricants. The formulations may be primarily administered in oral dose forms, such as tablets and capsules. It was also described that the use of granules brings better results than the direct pressing method, because granulation improves the flowability and compressibility of the active ingredient. In connection with the production of granules it is highlighted that on industrial scales only wet granulation brought acceptable flowability. Numerous possible excipients are listed in patent application No. US 2009/0110722A1. In addition to other substances, low substituted hydroxypropyl cellulose is mentioned among the disintegrants and binders that may be formulated with licarbazepine acetates. Nevertheless, in the patent claim stating the essence of the invention it is mentioned that in addition to licarbazepine acetate the pharmaceutical preparation also contains binder and disintegrant, where the disintegrant is present both intragranularly and extragranularly.

Researchers in India examined tablets containing eslicarbazepine acetate produced by wet granulation from the point of view of determining the difference between the dissolution properties of tablets produced using natural and synthetic binders (Formulation, evaluation and comparison of dissolution profiles of eslicarbazepine acetate immediate release tablets using natural binders against synthetic binder, International Journal of Pharmacy and Pharmaceutical Sciences; Vol 5, Suppl 4, 2013, 192-194.). They experienced that natural binders displayed better dissolution characteristics than tablets produced with the synthetic binder PVP (polyvinylpyrrolidone). All of the tablets produced for the study were produced from granules that, in addition to the active ingredient, contained sodium croscarmellose as disintegrant and, apart from this, various binders. No mention was made of low substituted hydroxypropyl cellulose, neither among the possible disintegrants nor among the possible binders.

International patent application No. WO 2017/103876 A1 describes an eslicarbazepine preparation containing a high active ingredient content, and a method for its production, in the case of which the disintegrant and/or the binder is only in the granules of the tablet or only between the granules. Eslicarbazepine acetate is substantially mentioned as the active ingredient. Low substituted hydroxypropyl cellulose is not mentioned among the possible binders, however, it is mentioned among the disintegrants.

According to the state of the art every concrete example relating to pharmaceutical preparations containing the active ingredient eslicarbazepine acetate contains a disintegrant and a binder as well, which according to the state of the art are different chemical substances.

It is known to a person skilled in the art that when producing tablets and capsules in the case of active ingredients that have a small particle size and that do not flow on their own and, as a result, cannot be capsuled or tableted in mechanical apparatuses, during the granulation process the binder facilitates the formation of larger particles or granules from the primary particles, that have good flow properties. In addition, in the case of tablets the binders also ensure appropriate hardness of the tablets during pressing.

The use of L-HPC (low-substituted hydroxypropyl cellulose) in pharmaceutical preparations as a substance promoting disintegration (disintegrant) was first approved in Japan in 1977.

The more important characteristics of L-HPC are as follows:
- excellent compatibility with active ingredients,
- disintegration into smaller particles, thereby facilitating better dissolution,
- prevents lamination (capping) of tablets,
- facilitates pellet extrusion.

Although their CAS number (Chemical Abstract number) is the same, L-HPC differs significantly from HPC (i.e. hydroxypropyl cellulose). There are separate articles for L-HPC and HPC in the pharmacopoeias. HPC is usually used as a binder in solid pharmaceutical forms. While in the case of HPC there is a large number of hydroxypropoxy groups on the cellulose frame, there are significantly fewer in the case of L-HPC. HPC is soluble in water, while L-HPC is substantially insoluble. An aqueous solution of HPC is commonly used for granulation. Due to it swelling in water L-HPC is an effective substance in promoting disintegration, which is not the case with HPC. As L-HPC can be easily pressed, solid tablets may be pressed from a dry mixture of it with the active ingredients. (Shinetsu brochure 05.8/1000)

L-HPC occurs in various morphological forms. Within the scope of the present specification differentiation is made between the fibrous and non-fibrous forms according to the shape of the particles. The latter may consist of particles micronized to various degrees.

### Brief description of the invention

On the basis of the above a general problem with presently known oral pharmaceutical preparations containing eslicarbazepine acetate is that due to the use of at least two excipients their manufacture includes complex, time-consuming processes. An example of such a problem is the measuring of the binders and disintegrants in several stages, their separate granulation, and homogenisation in several steps, and optionally, the preparation of an aqueous solution of the binder. Therefore, in spite of the quality of the currently marketed tablets is appropriate and they are suitable for the treatment of patients, their production methods may not be regarded as optimal.

Due to the abovementioned daily dose of 800 to 1600 mg in an oral eslicarbazepine acetate preparation (e.g. tablet, capsule), the active ingredient takes up a substantial proportion of it, therefore, in order for the preparation to have an optimal size it is necessary to minimise the amount of excipients as far as possible. Therefore, in the interest of patient compliance the formulation of the tablets and capsules must be performed with the use of as little excipient as possible.

Therefore, it is necessary to elaborate an eslicarbazepine acetate composition that may be used in a tablet or capsule that only contains one excipient in addition to the active ingredient (not including the shell in the case of capsule and the lubricant in the case of tablets) and that may be produced in a simpler, quicker and more economical way than in the case of the current methods.

During the investigations aimed at realising the above objectives it was found, surprisingly so for the person skilled in the art, that the desired objectives may be achieved by formulating eslicarbazepine acetate with low-substituted hydroxypropyl cellulose (hereinafter: L-HPC). Namely, using water as the wetting agent granules containing eslicarbazepine acetate suitable for producing tablets or capsules may be produced with the use of L-HPC. In accordance with the present invention low-substituted hydroxypropyl cellulose (L-HPC) is an excipient that performs the role of both binder and disintegrant by itself as a sole excipient and is suitable for producing granules containing eslicarbazepine acetate, thereafter from which capsules suitable for oral administration or tablets, with the addition of a lubricant, may be formulated.

In other words, the basis of the invention is the surprising recognition that the L-HPC in pharmaceutical preparations containing the active ingredient eslicarbazepine acetate does not only act as a disintegrant facilitating the appropriate degree of disintegration, but also as a binder displaying preferred binding properties, i.e. it performs the role of both disintegrant and binder as a single excipient. In a mixture containing eslicarbazepine acetate the L-HPC adapts the mixture so that granules with a suitable grain size may be produced from it. Furthermore, tablets made from these granules will have appropriate hardness, and the tablets and capsules formed from these granules will quickly disintegrate in an aqueous medium, i.e. after swallowing the medicine, as a result of the L-HPC and thereby ensure that the active ingredient is quickly released. It is stated here once again that it is desirable for the active ingredient to be released as quickly as possible in the case of treating epilepsy in the interest of achieving a fast effect.

The amount of the single excipient used as both disintegrant and binder, i.e. the necessary amount of L-HPC is less than if a separate disintegrant and binder were used, therefore, at a given dose, the tablets obtained will be smaller, which promotes better patient compliance.

Therefore, in accordance with the above, the present invention relates to granules that contain eslicarbazepine acetate and L-HPC, with the provisio that other ingredients can only be excipients that do not have any binding or disintegrating effect.

Also, the present invention relates to granules that essentially consist of eslicarbazepine acetate and L-HPC.

According to a preferred embodiment of the present invention the amount of L-HPC is at least 4% of the total mass of the granules.

According to a preferred embodiment of the invention the hydroxypropoxy content of the L-HPC in the granules is between 10% and 15%, and preferably 8%, 11% or 14%.

According to a preferred embodiment of the invention the shape of the L-HPC in the granules is non-fibrous.

According to a very preferred embodiment of the invention the hydroxypropoxy content of the L-HPC in the granules is 8% or 11% and the shape of the particles is non-fibrous.

The object of the present invention also relates to a method for the production of the granules according to the above, which method contains the following steps:
a1) eslicarbazepine acetate powder and L-HPC powder are mixed together,
b1) the powder mixture obtained in step a1) is granulated with water; or
a2) L-HPC powder is mixed with water in order to prepare an aqueous dispersion,
b2) eslicarbazepine acetate powder is granulated with the aqueous dispersion obtained in step a2);
or
a3) the eslicarbazepine acetate powder and a part of the L-HPC powder are mixed,
b3) the other part of the L-HPC powder is mixed with water to prepare an aqueous dispersion,
c3) the powder mixture obtained in step a3) and the aqueous dispersion obtained in step b3) are granulated.

The object of the present invention also relates to eslicarbazepine acetate tablets, which contain granules containing or consisting of eslicarbazepine acetate and L-HPC and a lubricant. The tablets preferably contain 200 mg to 1600 mg, more preferably 200 mg to 800 mg, most preferably 200 mg, 400 mg, 600 mg or 800 mg eslicarbazepine acetate. The lubricant is preferably magnesium stearate.

The object of the present invention also relates to eslicarbazepine acetate capsules that contain granules containing or consisting of eslicarbazepine acetate and L-HPC. The capsule preferably contains 200 mg to 400 mg eslicarbazepine acetate.

Furthermore, the object of the invention relates also to the use of the granules according to the above for the preparation of tablets or capsules.

Furthermore, the object of the invention also relates to the use of the granules according to the above for the production of a pharmaceutical preparation for the treatment of partial epileptic seizures, where the pharmaceutical preparation is preferably a tablet or a capsule.

### Detailed description of the invention

As described above, the essence of the present invention is that tablets and capsules containing eslicarbazepine acetate may be produced from granules that consist of eslicarbazepine acetate and L-HPC, as L-HPC is suitable well in such a preparation as both binder and disintegrant. As a single excipient is used in addition to the active ingredient, the granules according to the invention, and so the pharmaceutical preparation made from these, may be produced more quickly and cheaply as compared to the oral pharmaceutical preparations containing eslicarbazepine acetate currently known.

Unless otherwise stated % in the present specification is understood to mean mass percent (mass%), and in the case of ratios it is understood to mean the mass ratios of the individual components.

In the scope of the present specification if a numerical value is given, this is understood to mean that the last digit of the given number shows the precision of the given value according to the rounding-up rules. In other words, for example, 8% is understood to include all the values in the range 7.5% to 8.4%.

In the context of the present invention those hydroxypropyl celluloses are called L-HPC that contain 5.0 mass% to 16.0 mass% hydroxypropoxy groups with respect to dry material (USP Stage 4 Harmonization Official May 1, 2019). In the context of the present invention HPC may not be used as an excipient. Those hydroxypropyl celluloses are called HPC that contain 53.4 mass% to 80.5 mass% hydroxypropoxy groups with respect to dry material (USP, Stage 6 Harmonization Official December 1, 2014).

The L-HPCs are classed into various types depending on the degree of substitution (i.e. hydroxypropoxy content), particle shape and particle size. (Application Studies of L-HPC and HPCMCAS for Pharmaceutical Dosage Forms - Update-ExcipientFest Americas 2012. San Juan). (Table 1).

**Table 1: The characteristics of the various types of L-HPCs**

| Type | Mean particle size, D(50)* (µm) | D(90)**/ D(50) | Particle shape | Hydroxypropoxy content (%) |
|---|---|---|---|---|
| LH-11 | 50 | - | Most fibrous | 11 |
| LH-21 | 45 | 3.0 | Moderately fibrous | 11 |
| LH-22 | 45 | - | Moderately fibrous | 8 |
| LH-31 | 20 | - | Non-fibrous | 11 |
| LH-32 | 20 | - | Non-fibrous | 8 |
| LH-B1 | 50 | - | granulated | 11 |
| NBD-020 | 45 | 2.2 | Non-fibrous | 14 |
| NBD-021 | 45 | 2.2 | Non-fibrous | 11 |
| NBD-022 | 45 | 2.2 | Non-fibrous | 8 |

| | | | | |
|---|---|---|---|---|
| *D(50) this is the particle size that 50% of the particles are smaller than **D(90) this is the particle size that 90% of the particles are smaller than | | | | |

In the course of our investigations the suitability of the LH-21, LH-31, LH-32, LH-B1, NBD-020 and NBD-022 types L-HPC in preparations containing eslicarbazepine acetate was tested. During the tests the eslicarbazepine acetate powder was granulated with an aqueous suspension of L-HPC, thereby producing the granules according to the invention.

According to the present invention essentially all of the tested types of L-HPC may be used for producing the granules according to the invention, however, the non-fibrous types (see Table 1) are primarily preferred as the viscosity of their aqueous dispersions is lower, so they may be dispersed in less water, and so they are better for use in granulation. Among the non-fibrous L-HPCs those types with a more homogenous particle distribution are even more preferable. The lower the D90/D50 value characteristic of particle size distribution, the more homogenous the particle distribution. From this point of view the NDB types with a particle distribution of around 2.2 are preferred. Among the L-HPCs consisting of non-fibrous particles the L-HPC named NBD-022 containing 8% hydroxypropoxy groups proved to be particularly suitable.

In other words, the object of the invention relates to granules containing the known eslicarbazepine acetate and the known L-HPC, from which tablets and capsules that suitable as medicine can be manufactured, which medicines are suitable for treating partial epileptic seizures.

The production of the granules, i.e. granulation belongs to the obligatory knowledge of the person skilled in the art. During granulation the starting materials are homogenised, then in the case of wet granulation they are aggregated with a suitable granulation liquid, the wet aggregate is dried, then milled by being pressed through a sieve with the appropriate mesh size. In the case of dry granulation, the homogenised powder mixture is compressed into tablets or flat large disks, otherwise known as briquettes, which are then milled to produce the granules. Knowledge relating to this is discussed in detail in university textbooks and handbooks dealing with pharmaceutical production. E.g. Piroska Revesz (editor) Pharmaceutical Technology, 2009, JATEPress, 265-274; Dr Attila Dévay, The Theory and Practice of Pharmaceutical Technology, 2013, University of Pécs, 355-387; Pharmaceutical Dosage Forms: Tablets, Marcel Dekker, 1989, 2nd edition, volume 1, 148-151.

Therefore, the granules according to the invention contain eslicarbazepine acetate and L-HPC. The L-HPC plays the role of both binder and disintegrant in the granules. Optionally the granules according to the invention may also contain excipients that do not have any binding or disintegrating effect, such as pH-adjusting excipients, flavourings and colorants, stabilisers, etc.

The invention also relates to granules that substantially consist of eslicarbazepine acetate and L-HPC. Further excipients apart from L-HPC in addition to the necessarily large dose of eslicarbazepine acetate would increase the size of the oral pharmaceutical preparation to be produced from these granules, which would have a negative effect on patient compliance. However, it is obvious for the person skilled in the art that the granules according to the invention substantially consisting of eslicarbazepine acetate and L-HPC may also contain some water, as the granules are preferably produced using wet granulation, then the wet granules are dried to the optimal moisture content for tableting. This moisture content is usually between 0.1-6%, which, according to our experience, in the case of granules consisting of eslicarbazepine acetate and L-HPC is 0.5-4.0%. Furthermore, it is also obvious for a person skilled in the art that in addition to water the granules according to the invention substantially consisting of eslicarbazepine acetate and L-HPC may also contain contaminants that are not considered to be pharmaceutical excipients or active ingredients.

The L-HPC content of the granules according to the present invention is preferably at least 4% of the total mass of the granules. This amount of L-HPC ensures the granules have the appropriate formulation properties. A 4% L-HPC content substantially means an eslicarbazepine acetate: L-HPC mass ratio of 28:1.

An important technical parameter of the L-HPCs used in the granules according to the invention is their hydroxypropoxy content (see, for example, Table 1). In the course of our investigations, it was found that L-HPCs with a hydroxypropoxy content between 5% and 16% are suitable for producing the granules according to the invention, within these the L-HPCs with a hydroxypropoxy content of between 10% and 15% proved to be preferable. According to our investigations those L-HCPs were the most suitable for the production of the granules according to the invention that have a hydroxypropoxy content of 8%, 11% or 14%.

The granules according to the invention can be produced using wet granulation. The pharmaceutical industry granulation processes are well known to the person skilled in the art (see above). There are three methods that may be followed during the production of the granules according to the present invention:
1) eslicarbazepine acetate powder and L-HPC powder are mixed together and this powder mixture is granulated with water in the conventional way (aggregated, dried, milled);
2) firstly, an aqueous dispersion is produced from the powdered L-HPC by adding water, and then the eslicarbazepine acetate powder is granulated with this in the conventional way (aggregated, dried, milled);
3) eslicarbazepine acetate powder and a part of the L-HPC powder are mixed, the other part of the L-HPC powder is mixed with water to produce an aqueous dispersion, then the powder mixture is granulated with the aqueous dispersion in the conventional way (aggregated, dried, milled).

The granules according to the invention are preferably produced with the above method 2) or 3), as in these cases the use of 4% L-HPC with respect to the mass of the granules is sufficient, while in the case of the implementation of the first method it is necessary to use at least 20% L-HPC.

The object of the invention does not only relate to granules as a pharmaceutical preparation, but also to other oral pharmaceutical preparations produced from such granules. Such other oral pharmaceutical preparations according to the invention are preferably single dose preparations the active ingredient content of which is approximately between 200 mg and 1600 mg, more preferably between 200 mg and 800 mg, most preferably 200 mg, 400 mg, 600 mg or 800 mg. Such other oral pharmaceutical preparations may be tablets or capsules.

In order to produce the tablets according to the invention an appropriate amount of lubricant is added to the given amount of granules according to the invention (for example, usually 0.25 to 5.0% in the case of magnesium stearate), then tablets are produced from this by pressing. The production of pressed tablets by wet granulation is known, e.g. according to that described in the following literature: Pharmaceutical Dosage Forms: Tablets, 2nd edition, Marcel Dekker 1989, volume 1, pages 131-193).

According to one of the most preferred embodiments of the tablet according to the invention, it contains 800 mg eslicarbazepine acetate. As it is the dose that has proven to be effective earlier in the treatment of partial epileptic seizures.

Lubricants are preferably used in order to reduce the friction occurring during the pressing of the tablets produced from the granules according to the invention. Any customary lubricant may be used for this purpose (e.g. magnesium stearate, calcium stearate, zinc stearate, stearic acid, glycerine mono- and distearates, hydrogenated vegetable oils, paraffin, silicon oil, etc.). Magnesium stearate is preferably used.

The test results relating to the tablets according to the invention have been summarised in Table 2.

**Table 2: The pharmaceutical technology parameters of tablets produced with L-HPC containing eslicarbazepine acetate**

| **Test serial number** | 1 | 2 | 3 | 4 | 5 | 6 |
|---|---|---|---|---|---|---|
| eslicarbazepine acetate (mg) | 800 | 800 | 800 | 800 | 800 | 800 |
| magnesium stearate (mg) | 16 | 16 | 16 | 16 | 16 | 16 |
| L-HPC LH-21 (mg) | 60 | | | | | |
| L-HPC LH-31 (mg) | | 60 | | | | |
| L-HPC LH-B1 (mg) | | | 60 | | | |
| L-HPC NBD-020 (mg) | | | | 60 | | |
| L-HPC LH-32 (mg) | | | | | 60 | |
| L-HPC NBD-022 (mg) | | | | | | 60 |

| **Tablet parameters** | | | | | | |
|---|---|---|---|---|---|---|
| Total mass of tablet (mg) | 876 | 876 | 876 | 876 | 876 | 876 |
| Crush resistance (N) | 161 | 147 | 133 | 170 | 152 | 179 |
| Ph. Eur,9, 2.9.8 | | | | | | |
| Disintegration time (s) | 20 | 20 | 20 | 37 | 30 | 40 |
| Ph. Eur,9, 2.9.1 | | | | | | |
| Dissolution 30 minutes % | 79.5 | 84.2 | 77.2 | 80.7 | 80.5 | 81.2 |
| Ph. Eur. 9.2.9.3 (mixing device, 100 rpm, pH 4.5) | | | | | | |

The data in Table 2 show that the active ingredient content (91.3%) of the tablets produced with the lubricant magnesium stearate from the granules containing eslicarbazepine acetate and various types of L-HPC is higher as compared to the preparations known according to the state of the art (80-90%), furthermore the pharmaceutical technology characteristics of the tablets meet the standard requirements as follows:
- the crush resistence of the tablets is above 130 N, as opposed to the customary minimum of 40 N (4 kg) (Pharmaceutical Manufacturing Handbook, Production and Processes, SHAYNE COX GAD, PH.D., D.A.B.T., 2008 by John Wiley & Sons, Inc. page 928);
- the disintegration time of the tablets is within 1 minute as opposed to the customarily prescribed 15 minutes (Ph. Eur. 9 Page 886);
- the dissolution of the active ingredient in 30 minutes measured according to Ph. Eur. Procedure number 9.2.9.3 significantly exceeds 60% (minimum requirement according to claim 1 of patent specification US 8,372,431 B2).

During our investigations it was found that as the single excipient, apart from the lubricant, in the eslicarbazepine acetate tablet produced from the granules according to the invention the L-HPC is exceptionally suitable for the production of a preparation with a high active ingredient content and fast dissolution, as this excipient is suitable on its own for performing the functions of both binder and disintegrant. This dual effect of L-HPC was experienced in both the cases of granulation in powder form with water, and in aqueous dispersion used as granulation liquid.

The process followed during the usual production of a capsule according to the invention is that optionally lubricant is mixed with an appropriate amount of granules according to the invention and the mixture obtained in this way is filled into a capsule. According to the present invention the use of a lubricant is not necessary in the case of capsules.

The capsule is a two-part hard capsule, with wall material of gelatine or HPMC (hydroxypropyl methylcellulose).

The capsules according to the invention contain 200 mg to 400 mg eslicarbazepine acetate per capsule.

In the interest of reducing the total mass of the oral pharmaceutical preparation the mass ratio of eslicarbazepine acetate and L-HPC in the granules may even be 25:1, i.e. the active ingredient content of the preparation may even be as much as 96 mass%. Nevertheless, because of the desired high dose in order to achieve at least 90% active ingredient content, the mass ratio of eslicarbazepine acetate and L-HPC is preferably at least 10:1. On the other hand, the production of the oral pharmaceutical preparations according to the present invention is significantly simpler as they contain fewer components, and these do not have to be divided in the granules, and outside of the granules, which in addition to the less work being involved, this may also result in better uniformity of the active ingredient content.

In accordance with the above the present invention also relates to the use of the granules according to the invention for the production of further oral pharmaceutical preparations, such as tablets or capsules. The granules according to the invention themselves are to be considered as a pharmaceutical preparation, which may be used on its own, but the granules may also be used at the site of their production or remotely from it, immediately following production, or later on in time for the production of further oral pharmaceutical preparations, such as tablets or capsules, for example.

The object of the invention also relates to a use of the granules according to the present invention during which a medicine for the treatment of partial epileptic seizures is produced. It was described above that eslicarbazepine acetate proved to be effective as a pharmaceutical active ingredient in this indication. Therefore, a pharmaceutical preparation produced from the granules according to the invention made from this active ingredient is also suitable for use in the aforementioned indication. The pharmaceutical preparation according to the invention is preferably a tablet or capsule.

Further details of the invention are described in the following examples without restricting the invention to the examples.

### Example 1: Granulation with an aqueous suspension of L-HPC

120 g eslicarbazepine acetate is filled into the material container of a Procept 4M8 eddy current granulator. The material is homogenised for 1 minute, then during constant stirring a suspension of 9 g L-HPC LH-21 and 6 g water is added and after this it is granulated by kneading for 10 minutes. The wet granules are dried to a moisture content of under 1% in a Procept 4M8 fluid bed dryer. The granules are passed through a sieve with a mesh size of 0.8 mm, 2.4 g magnesium stearate is mixed into it, then using a Korsch EX0 tableting machine and a 19x8 mm oblong shape punch die, tablets with an average mass of 876 mg are pressed.

The tablets contain 800 mg eslicarbazepine acetate (91.3%).

The method is repeated with the following types of L-HPC: L-HPC LH-31, L-HPC LH-32, L-HPC LH-B1, L-HPC NBD-020 and LHPC NBD-022.

The pharmaceutical technology parameters of the tablets produced in this way are given in the above Table 2.

### Example 2: Granulation with water

100 g eslicarbazepine acetate and 25 g L-HPC LH-B1 are filled into the material container of a Procept 4M8 eddy current granulator. The material is homogenised for 3 minutes, then after adding 140 g water the material is granulated by kneading for 10 minutes, following this it is dried in a Procept 4M8 fluid bed dryer. The granules are sieved, 2 g magnesium stearate is admixed, then using a Korsch EX0 tableting machine and a 19x8 mm oblong shape punch die, tablets with an average mass of 750 mg are pressed.

The tablets contain 600 mg eslicarbazepine acetate.

The crush resistance of the tablets is 150 N, their disintegration time is 1 minute.

### Example 3: Investigation of the effect of the amount of L-HPC

The production of the granules is performed according to example 1, but 7.5 g or 4.8 g L-HPC NBD-020 are then consecutively used in the granulation fluid.

The eslicarbazepine acetate : L-HPC ratio in these cases is 16:1, and 25:1.

After adding 1.2 g magnesium stearate to the granules the active ingredient content of the tablets is 93.2%, and 95.2%.

The crush resistance of the tablets is 175 N, and 159 N. The disintegration time of the tablets is 43 s, and 9 min. As expected the reduction of the amount of L-HPC involved the reduction of the crush resistance of the tablets and the increase of the disintegration time, which are negative effects.

In the case of the use of an amount of L-HPC smaller than this (e.g. in the case of a 33:1 ratio) the mechanical strength and the disintegration time of the tablets were no longer acceptable. The crush resistance dropped to under 40 N, and the disintegration time increased to more than 60 minutes.

### Example 4: Eslicarbazepine acetate 400 mg capsule and tablet production

120 g eslicarbazepine acetate is filled into the material container of a Procept 4M8 eddy current granulator. The material is homogenised for 1 minute, then during constant stirring a suspension of 9 g L-HPC NBD-020 and 66 g water is added, and after this it is granulated by kneading for 10 minutes. The wet granules are dried to a moisture content of approximately 1% in a Procept 4M8 fluid bed dryer. The granules are sieved and divided into two parts.

430 mg from one half of the granules is filled into size "0" capsules without the addition of magnesium stearate. The dissolution of the capsules in 30 minutes: 90.2%.

1.2 g magnesium stearate is added to the other half of the granules and then using a 12 mm diameter lens-shaped punch die tablets are pressed with an average mass of 438 mg in a Korsch EX0 tableting machine.

The capsules contain 400 mg eslicarbazepine acetate (91.3%). The dissolution of the capsules in 30 minutes is 93.5%. The smaller dose (400 mg) results in higher dissolution as compared to the other examples. The crush resistance of the tablets is 96 N, friability 0.6%, disintegration time 0.6 minutes, dissolution in 30 minutes is 96.5%.

### Example 5: Production of eslicarbazepine acetate tablets using a pilot plant tableting machine

480 g eslicarbazepine acetate is filled into the material container of a Procept 4M8 eddy current granulator. The material is homogenised for 1 minute, then during constant stirring a suspension of 40 g L-HPC NBD-020 and 300 g water is added, and after this it is granulated by kneading for 10 minutes. The wet granules are dried to a moisture content of approximately 1% in a Procept 4M8 fluid bed dryer using air at a temperature of 60 °C. The granules are passed through a sieve with a mesh size of 0.8 mm, 9.6 g magnesium stearate is admixed to it, then using a Wynka rotary tablet press and a 19x8 mm oblong shape punch die tablets are pressed with an average mass of 876 mg.

The tablets contain 800 mg eslicarbazepine acetate (91.3%).

The pharmaceutical technology parameters of the tablets:

| Tablet mass (mg) | | 876 mg |
|---|---|---|
| Crush resistance (N) | | 196 N |
| Ph. Eur. 9, 2.9.8 | | |
| Disintegration time (s) | | 90 |
| Ph. Eur. 9, 2.9.1 | | |
| Friability* | | 0.04% |
| Ph. Eur. 9, 2.9.7 | | |
| Dissolution (30 minutes) % | | 77.6% |
| | Ph. Eur. 9, 2.9.3 stirring apparatus, 100 rpm, pH 4.5 buffer | |

| | | |
|---|---|---|
| *Friability: the mass loss of the tablets that occurs due to the effect of 100 drops in an apparatus according to the prescriptions of the pharmacopoeia. General requirement relating to friability: max. 1%. (Ph. Eur.9, 2.9.7.) | | |

Consequently, tablets with very good mechanical properties and a very short disintegration time could be produced using a pilot plant rotary tablet press from the granules according to the invention containing the L-HPC and magnesium stearate.

### Example 6: Granulation of a powder mixture containing eslicarbazepine acetate and L-HPC

560 g eslicarbazepine acetate and 31.55 g L-HPC NBD-022 are filled into the material container of a Procept 4M8 eddy current granulator. The material is homogenised for 3 minutes, then after adding a suspension containing 10.5 g L-HPC NBD-022 and 300 g water it is granulated by kneading for 10 minutes. The wet granules are dried to a moisture content of approximately 1% in a Procept 4M8 fluid bed dryer using air at a temperature of 60 °C. The granules are passed through a sieve with a mesh size of 0.8 mm, 8.8 g magnesium stearate is admixed to it then using a Wynka rotary tablet press and a 19x8 mm oblong shape punch die tablets are pressed with an average mass of 876 mg.

The tablets contain 800 mg eslicarbazepine acetate. The active ingredient content of the tablets is 91.6%.

The pharmaceutical technology parameters of the tablets:

| | | |
|---|---|---|
| Crush resistance (N) | | 156 |
| Ph. Eur. 9, 2.9.8 | | |
| Disintegration time (s) | | 36 |
| Ph. Eur. 9, 2.9.1 | | |
| Friability (%) | | 0.11 |
| Ph. Eur. 9, 2.9.7 | | |
| Dissolution (30 minutes) % | | 79.0 |
| | Ph. Eur. 9, 2.9.3 stirring apparatus, 100 rpm, pH 4.5 buffer | |

Consequently, tablets of the appropriate quality may even be produced from the granules according to the invention containing L-HPC and magnesium stearate in a pilot plant tablet press if a part of the L-HPC is mixed in powder form with the active ingredient, and the granulation is performed with a suspension made from the remaining part of the L-HPC. The advantage of this solution is that as a result of the division of the amount of L-HPC the viscosity of the granulation suspension may be reduced, which facilitates the performance of the operations (suspension production, addition, granulation), and enables a reduction of the amount of the granulation liquid, which results in a reduction of the energy required to dry the granules and the operation time.

### Example 7: Pilot production of granules containing eslicarbazepine acetate and L-HPC

40.00 kg eslicarbazepine acetate and 2.25 kg L-HPC NBD-022 are filled into the material container of a Zanchetta 200 eddy current granulator. The material is homogenised for 3 minutes, then after adding a suspension of 0.75 kg L-HPC NBD-022 and 22.00 kg water it is granulated by kneading for 10 minutes. The wet granules are dried to a moisture content of approximately 1% in a Glatt WSG 30 fluid bed dryer using air at a temperature of 60 °C. The granules are passed through a sieve with a mesh size of 0.8 mm, 0.80 kg magnesium stearate is admixed to it then using a Wynka rotary tablet press and a 19x8 mm oblong shape punch die tablets are pressed with an average mass of 876 mg.

The tablets contain 800 mg eslicarbazepine acetate. The active ingredient content of the tablets is 91.6%.

In order to verify the reproducibility of the process the above production was repeated twice.

The pharmaceutical technology parameters of the tablets:

| Parameter | | Production 1 | Production 2 | Production 3 |
|---|---|---|---|---|
| Crush resistance (N) | | 132 | 130 | 147 |
| Ph. Eur. 9, 2.9.8 | | | | |
| Disintegration time (s) | | 66 | 50 | 50 |
| Ph. Eur. 9, 2.9.1 | | | | |
| Friability (%) | | 0.31 | 0.21 | 0.15 |
| Ph. Eur. 9, 2.9.7 | | | | |
| Dissolution (30 minutes) % | | 77.0 | 78.4 | 76.5 |
| | Ph. Eur. 9, 2.9.3 stirring apparatus, 100 rpm, pH 4.5 buffer | | | |

Consequently, tablets of the appropriate quality may be reproducibly manufactured even in the case of a pilot production amount of approximately 50,000 tablets.

## Claims

1. Granules, **characterised by that** they contain eslicarbazepine acetate and L-HPC, with the provisio that other ingredients can only be excipients that do not have any binding or disintegrating effect.

2. Granules, **characterised by that** they consist substantially of eslicarbazepine acetate and L-HPC.

3. Granules according to claim 1 or 2, **characterised by that** the amount of L-HPC is at least 4% of the total mass of the granules.

4. Granules according to any of claims 1 to 3, **characterised by that** the hydroxypropoxy content of the L-HPC in the granules is 8% or between 10% and 15%, and preferably 8%, 11% or 14%.

5. Granules according to any of claims 1 to 4, **characterised by that** the shape of the L-HPC particles is non-fibrous, preferably their D90/D50 value characteristic of particle size distribution is approximately 2.2.

6. Method for the production of the granules according to any of claims 1 to 5, **characterised by that** the method contains the following steps:
a1) eslicarbazepine acetate powder and L-HPC powder are mixed together,
b1) the powder mixture obtained in step a1) is granulated with water; or
a2) L-HPC powder is mixed with water in order to prepare an aqueous dispersion,
b2) eslicarbazepine acetate powder is granulated with the aqueous dispersion obtained in step a2);
or
a3) the eslicarbazepine acetate powder and a part of the L-HPC powder are mixed,
b3) the other part of the L-HPC powder is mixed with water to prepare an aqueous dispersion,
c3) the powder mixture obtained in step a3) and the aqueous dispersion obtained in step b3) are granulated.

7. Tablet containing eslicarbazepine acetate, **characterised by that** it contains the granules according to any of claims 1 to 5 and a lubricant.

8. Tablet according to claim 7, **characterised by that** it contains 200 mg to 1600 mg, more preferably 200 mg to 800 mg, most preferably 200 mg, 400 mg, 600 mg or 800 mg eslicarbazepine acetate.

9. Tablet according to claim 7 or 8, **characterised by that** the lubricant is magnesium stearate.

10. Capsule containing eslicarbazepine acetate, **characterised by that** it contains the granules according to any of claims 1 to 5.

11. Capsule according to claim 10, **characterised by that** it contains 200 mg to 400 mg eslicarbazepine acetate.

12. The use of the granules according to any of claims 1 to 5 for the production of tablets or capsules.

13. The use of the granules according to any of claims 1 to 5 for the production of a pharmaceutical preparation for the treatment of partial epileptic seizures.

14. The use according to claim 13, **characterised by that** the pharmaceutical preparation is preferably a tablet or a capsule.

## Patentansprüche

1. Granulat, **dadurch gekennzeichnet, dass** es Eslicarbazepinacetat und L-HPC enthält, mit der Maßgabe, dass andere Inhaltsstoffe nur Hilfsstoffe, die keine bindende oder zersetzende Wirkung haben, sein können.

2. Granulat, **dadurch gekennzeichnet, dass** es im Wesentlichen aus Eslicarbazepinacetat und L-HPC besteht.

3. Granulat nach Anspruch 1 oder 2, **dadurch gekennzeichnet, dass** die Menge an L-HPC mindestens 4 % der Gesamtmasse des Granulats beträgt.

4. Granulat nach einem der Ansprüche 1 bis 3, **dadurch gekennzeichnet, dass** der Hydroxypropoxygehalt des L-HPC im Granulat 8 % oder zwischen 10 % und 15 % und vorzugsweise 8 %, 11 % oder 14 % beträgt.

5. Granulat nach einem der Ansprüche 1 bis 4, **dadurch gekennzeichnet, dass** die Form der L-HPC-Partikel nicht faserig ist, vorzugsweise ihr für die Partikelgrößenverteilung charakteristischer D90/D50-Wert etwa 2,2 beträgt.

6. Verfahren zur Herstellung des Granulats nach einem der Ansprüche 1 bis 5, **dadurch gekennzeichnet, dass** das Verfahren die folgenden Schritte aufweist:
a1) Eslicarbazepinacetat-Pulver und L-HPC-Pulver werden zusammengemischt,
b1) die in Schritt a1) erhaltene Pulvermischung wird mit Wasser granuliert; oder
a2) L-HPC-Pulver wird mit Wasser gemischt, um eine wässrige Dispersion herzustellen,
b2) Eslicarbazepinacetat-Pulver wird mit der in Schritt a2) erhaltenen wässrigen Dispersion granuliert;
oder
a3) das Eslicarbazepinacetat-Pulver und ein Teil des L-HPC-Pulvers werden gemischt,
b3) der andere Teil des L-HPC-Pulvers wird mit Wasser gemischt, um eine wässrige Dispersion herzustellen,
c3) die in Schritt a3) erhaltene Pulvermischung und die in Schritt b3) erhaltene wässrige Dispersion werden granuliert.

7. Eslicarbazepinacetat enthaltende Tablette, **dadurch gekennzeichnet, dass** sie das Granulat nach einem der Ansprüche 1 bis 5 und ein Gleitmittel enthält.

8. Tablette nach Anspruch 7, **dadurch gekennzeichnet, dass** sie 200 mg bis 1600 mg, besonders bevorzugt 200 mg bis 800 mg, am meisten bevorzugt 200 mg, 400 mg, 600 mg oder 800 mg Eslicarbazepinacetat enthält.

9. Tablette nach Anspruch 7 oder 8, **dadurch gekennzeichnet, dass** das Schmiermittel Magnesiumstearat ist.

10. Eslicarbazepinacetat enthaltende Kapsel, **dadurch gekennzeichnet, dass** sie das Granulat nach einem der Ansprüche 1 bis 5 enthält.

11. Kapsel nach Anspruch 10, **dadurch gekennzeichnet, dass** sie 200 mg bis 400 mg Eslicarbazepinacetat enthält.

12. Verwendung des Granulats nach einem der Ansprüche 1 bis 5 zur Herstellung von Tabletten oder Kapseln.

13. Verwendung des Granulats nach einem der Ansprüche 1 bis 5 zur Herstellung einer pharmazeutischen Zubereitung zur Behandlung partieller epileptischer Anfälle.

14. Verwendung nach Anspruch 13, **dadurch gekennzeichnet, dass** die pharmazeutische Zubereitung vorzugsweise eine Tablette oder eine Kapsel ist.

## Revendications

1. Granules, **caractérisés en ce qu'**ils contiennent de l'acétate d'eslicarbazépine et du L-HPC, à condition que les autres ingrédients ne puissent être que des excipients n'ayant aucun effet de liaison ou de désintégration.

2. Granules, **caractérisés en ce qu'**ils sont constitués essentiellement d'acétate d'eslicarbazépine et de L-HPC.

3. Granules selon la revendication 1 ou 2, **caractérisés en ce que** la quantité de L-HPC est d'au moins 4% de la masse totale des granules.

4. Granules selon l'une quelconque des revendications 1 à 3, **caractérisés en ce que** la teneur en hydroxypropoxy du L-HPC dans les granules est de 8% ou comprise entre 10% et 15%, et de préférence de 8%, 11 % ou 14%.

5. Granules selon l'une quelconque des revendications 1 à 4, **caractérisés en ce que** la forme des particules de L-HPC est non fibreuse, de préférence leur valeur D90/D50 caractéristique de la distribution granulométrique est d'environ 2,2.

6. Procédé de production des granules selon l'une quelconque des revendications 1 à 5, **caractérisé en ce que** le procédé contient les étapes suivantes :
a1) la poudre d'acétate d'eslicarbazépine et la poudre de L-HPC sont mélangées ensemble,
b1) le mélange de poudre obtenu à l'étape a1) est granulé avec de l'eau ; ou
a2) la poudre de L-HPC est mélangée à de l'eau afin de préparer une dispersion aqueuse,
b2) la poudre d'acétate d'eslicarbazépine est granulée avec la dispersion aqueuse obtenue à l'étape a2) ;
ou
a3) la poudre d'acétate d'eslicarbazépine et une partie de la poudre de L-HPC sont mélangées,
b3) l'autre partie de la poudre de L-HPC est mélangée avec de l'eau pour préparer une dispersion aqueuse,
c3) le mélange de poudre obtenu à l'étape a3) et la dispersion aqueuse obtenue à l'étape b3) sont granulés.

7. Comprimé contenant de l'acétate d'eslicarbazépine, **caractérisé en ce qu'**il contient les granules selon l'une quelconque des revendications 1 à 5 et un lubrifiant.

8. Comprimé selon la revendication 7, **caractérisé en ce qu'**il contient de 200 mg à 1600 mg, plus préférablement de 200 mg à 800 mg, le plus préférablement 200 mg, 400 mg, 600 mg ou 800 mg d'acétate d'eslicarbazépine.

9. Comprimé selon la revendication 7 ou 8, **caractérisé en ce que** le lubrifiant est le stéarate de magnésium.

10. Capsule contenant de l'acétate d'eslicarbazépine, **caractérisée en ce qu'**elle contient les granules selon l'une quelconque des revendications 1 à 5.

11. Capsule selon la revendication 10, **caractérisée en ce qu'**elle contient de 200 mg à 400 mg d'acétate d'eslicarbazépine.

12. Utilisation des granules selon l'une quelconque des revendications 1 à 5 pour la production de comprimés ou de capsules.

13. Utilisation des granules selon l'une quelconque des revendications 1 à 5 pour la production d'une préparation pharmaceutique pour le traitement des crises d'épilepsie partielles.

14. Utilisation selon la revendication 13, **caractérisée en ce que** la préparation pharmaceutique est de préférence un comprimé ou une capsule.
